(19) Europäisches Patentamt
European Patent Office
Office européen des brevets

(11) Numéro de publication: **0 378 026 A1**

(12) # DEMANDE DE BREVET EUROPEEN

(21) Numéro de dépôt: **89403669.8**

(22) Date de dépôt: **28.12.89**

(51) Int. Cl.5: **C07C 69/743, A01N 53/00, C07C 61/40, C07F 7/08**

Revendications pour l'Etat contractant suivant: ES.

(30) Priorité: **29.12.88 FR 8817396**

(43) Date de publication de la demande: **18.07.90 Bulletin 90/29**

(84) Etats contractants désignés: **BE CH DE ES FR GB IT LI LU NL**

(71) Demandeur: **ROUSSEL-UCLAF**
**35, boulevard des Invalides**
**F-75007 Paris(FR)**

(72) Inventeur: **Benoit, Marc**
**Le Cannet Est - Pont de l'Etoile**
**F-13360 Roquevaire(FR)**
Inventeur: **Demoute, Jean-Pierre**
**Canta Cigalo**
**F-13390 Auriol(FR)**

(74) Mandataire: **Vieillefosse, Jean-Claude et al**
**Roussel-Uclaf 111, route de Noisy B.P. 9**
**F-93230 Romainville(FR)**

(54) **Nouveaux dérivés fluorés de l'acide 3-éthényl 2,2-diméthyl cyclopropane carboxylique, leur procédé de préparation, leur application comme pesticides et les compositions les renfermant.**

(57) L'invention concerne les composés de formule (I) :

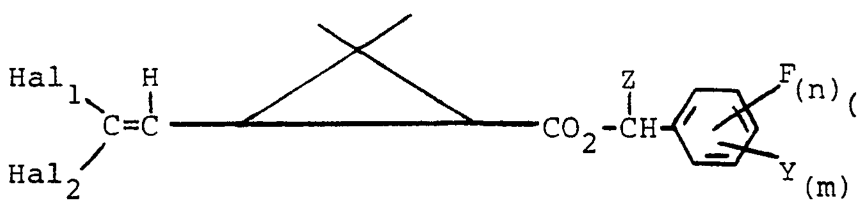
(I)

sous toutes les formes stéréoisomères possibles ainsi que les mélanges de ces stéréoisomères, dans lesquels :

- $Hal_1$ et $Hal_2$ différents l'un de l'autre représentent un atome de fluor, de chlore, de brome ou d'iode,
- Z représente un atome d'hydrogène, un radical $CH_3$, C≡N ou C≡CH,
- n représente un nombre entier pouvant varier de 1 à 5,
- m représente le nombre 5-n et,
- Y représente un atome d'hydrogène, d'halogène, un radical $CH_2$-C N, un hydroxy, un alkyle $(C_{1-8})$ éventuellement substitué, nitrile,

$\overset{O}{\overset{\|}{C}}$-O alkyl,

$\overset{O}{\overset{\|}{C}}$-alkyle, $(CH_2)m'$ O-alkyle, $(CH_2)m'$S alkyle, $(CH_2)m'$ N-$(alkyle)_2$ $(C_{1-12})$, m' représentant 0, 1, 2, 3 ou 4, un radical Si $(alkyle)_3$, alkyle représentant un alkyle $(C_{1-8})$ éventuellement substitué, O-aryle ou -$(CH_2)m'$-aryle, aryle représentant un aryle $(C_{6-14})$, le ou les substituants Y identiques ou différents, pouvant être en position quelconque sur le noyau phényle, leur procédé de préparation, leur application comme pesticides et les compositions les renfermant.

EP 0 378 026 A1

# Nouveaux dérivés fluorés de l'acide 3-éthényl 2,2-diméthyl cyclopropane carboxylique, leur procédé de préparation leur application comme pesticides et les compositions les renfermant.

La présente invention concerne de nouveaux dérivés fluorés de l'acide 3-éthényl 2,2-diméthyl cyclopropane carboxylique, leur procédé de préparation, leur application comme pesticides et les compositions les renfermant.

L'invention a pour objet sous toutes les formes stéréoisomères possibles ainsi que les mélanges de ces stéréoisomères les composés de formule (I)

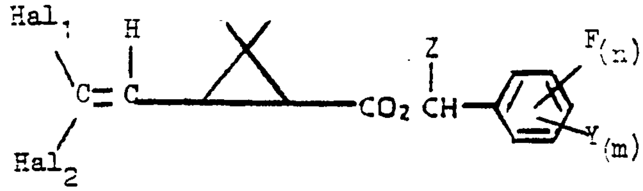

(I)

dans laquelle :

- $Hal_1$ et $Hal_2$ différents l'un de l'autre représentent un atome de fluor, de chlore, de brome ou d'iode,
- Z représente un atome d'hydrogène, un radical $CH_3$, C≡N ou C≡CH,
- n représente un nombre entier pouvant varier de 1 à 5,
- m représente le nombre 5-n et,
- Y représente un atome d'hydrogène, un atome d'halogène, un radical $CH_2$-C≡N, un radical hydroxy, un radical alkyle linéaire, ramifié, saturé ou insaturé éventuellement substitué renfermant jusqu'à 8 atomes de carbone un radical nitrile,

un radical

$\overset{O}{\overset{\|}{C}}$-O alkyle,

$\overset{O}{\overset{\|}{C}}$-alkyle, $(CH_2)m'$ O-alkyle, $(CH_2)m'$ S-alkyle, $(CH_2)m'$ N-$(alkyle)_2$, dans lequel m' représente le nombre 0, 1, 2, 3 ou 4 et renfermant jusqu'à 12 atomes de carbone, un radical Si $(alkyle)_3$, alkyle représente un radical alkyle linéaire ou ramifié, saturé ou insaturé, éventuellement substitué renfermant jusqu'à 8 atomes de carbone, un radical O-aryle ou -$(CH_2)m'$-aryle, aryle représentant un radical aryle renfermant jusqu'à 14 atomes de carbone, le ou les substituants Y identiques ou différents, pouvant être en position quelconque sur le noyau phényle.

Ainsi, $Hal_1$ peut représenter un atome de fluor, et $Hal_2$ un atome de chlore, de brome ou d'iode.

$Hal_1$ peut représenter un atome de chlore et $Hal_2$ un atome de brome ou d'iode.

$Hal_1$ peut représenter un atome de brome et $Hal_2$ un atome d'iode.

Dans la définition de Y, lorsqu'il s'agit de radicaux alkyles, il s'agit de préférence de radicaux méthyle, éthyle, propyle, isopropyle, n-butyle ou tertbutyle.

Lorsque Y représente un radical alkyle insaturé, il s'agit d'un radical éthylénique comme par exemple éthényle, propényle ou propadiényle ou d'un radical acétylénique comme par exemple le radical éthynyle ou propynyle.

Lorsque Y représente un radical alkyle substitué par un ou plusieurs groupements fonctionnels, on entend de préférence par groupement fonctionnel les atomes d'halogène, tels que le fluor ou le brome ; Y peut être par exemple un radical $CF_3$.

Lorsque Y représente un radical $(CH_2)m'$ O alkyle, ou $(CH_2)n'$ S alkyle, il s'agit de préférence du radical $OCH_3$ ou $SCH_3$.

Lorsque Y représente un radical O-aryle ou $(CH_2)m'$ aryle on entend de préférence par aryle le radical phényle.

L'invention a tout particulièrement pour objet les composés de formule ($I_A$) :

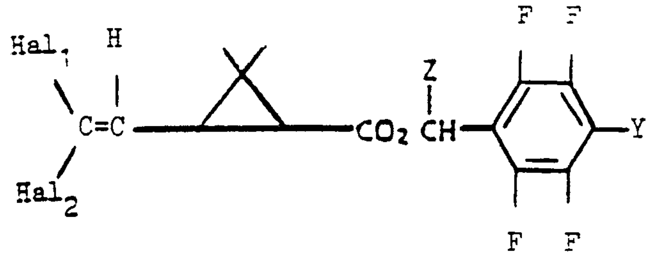

$(I_A)$

dans lesquels $Hal_1$, $Hal_2$, X, Z et Y conservent la même signification que précédemment.

Parmi les composés préférés, on peut citer en particulier les composés dans lesquels :

- Y représente un atome d'hydrogène, un atome d'halogène, un radical hydroxy, un radical alkyle linéaire, ramifié, saturé ou insaturé éventuellement substitué renfermant jusqu'à 8 atomes de carbone, un radical nitrile, un radical $(CH_2)m'$ O-alkyle, $(CH_2)m'$ S alkyle, $(CH_2)m'$ N-(alkyle)$_2$, dans lequel m' représente le nombre 0,1,2,3 ou 4 et renfermant jusqu'à 12 atomes de carbone un radical Si (alkyle)$_3$, alkyle représente un radical alkyle linéaire ou ramifié, saturé ou insaturé, éventuellement substitué renfermant jusqu'à 8 atomes de carbone, un radical O-aryle ou -$(CH_2)$m-aryle, aryle représentant un radical aryle renfermant jusqu'à 14 atomes de carbone.

Parmi les composés de l'invention préférés, on peut citer les composés dans lesquels $Hal_1$ et $Hal_2$ représentent l'un un atome de fluor, et l'autre un atome de chlore, ainsi que ceux dans lesquels $Hal_1$ et $Hal_2$ représentent l'un un atome de fluor et l'autre un atome de brome. On peut également citer les composés dans lesquels Z représente un atome d'hydrogène.

L'invention a notamment pour objet les composés dans lesquels Y représente un atome de fluor, un atome d'hydrogène ou encore un radical 2-propynyle, un radical 2-propényle ou un radical éthyle.

L'invention a tout particulièrement pour objet les composés dans lesquels la copule cyclopropanique est de structure 1Rcis.

L'invention a tout spécialement pour objet les composés dont la préparation est donnée dans la partie expérimentale et tout particulièrement les composés des exemples 1, 2, 3, 4, 5, 6, 10, 44 et 45.

L'invention a également pour objet un procédé de préparation des composés de formule I caractérisé en ce que l'on soumet en présence d'un agent d'estérification un acide de formule (II) :

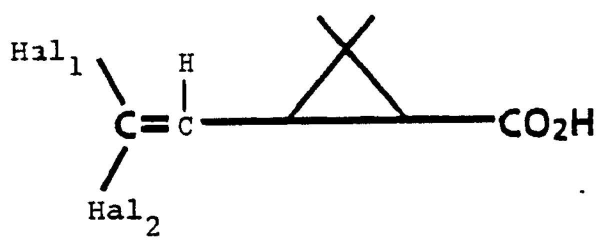

(II)

dans laquelle $Hal_1$ et $Hal_2$ conservent leur signification précédente, à l'action d'un alcool de formule (III) :

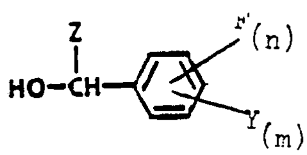

(III)

dans laquelle Y, Z, n et m conservent leur signification précédente pour obtenir le composé de formule I correspondant.

Les composés de formule II sont des produits connus de façon générale décrits par exemple dans le brevet français 2396006. Les composés de formule II de structure 1R trans sont préparés selon un procédé analogue au procédé de structure 1Rcis. Les acides décrits ci-après en "préparation" sont nouveaux et sont un objet de la présente invention.

Les composés de formule III sont des composés connus d'une manière générale ; ils peuvent être préparés par exemple selon les procédés décrits dans la demande de brevet européen 0031199, dans les

brevets américains 4370346, 4405640, dans le brevet anglais 2171994 dans British Crop Protection Conference Pest and Desease 1986 page 199 ou encore dans la demande de brevet européen 0281439.

Les composés de formule I présentent d'intéressantes propriétés qui permettent leur utilisation dans la lutte contre les parasites. Il peut s'agir par exemple de la lutte contre les parasites des végétaux, qu'il s'agisse des parasites du sol ou des parties aériennes, les parasites des locaux et les parasites des animaux à sang chaud.

C'est ainsi que l'on peut utiliser les produits de l'invention pour lutter contre les insectes, les nématodes et les acariens parasites des végétaux et des animaux.

L'invention a notamment pour objet l'application des composés de formule I à la lutte contre les parasites des végétaux, les parasites des locaux et les parasites des animaux à sang chaud.

Les produits de formule I peuvent aussi être utilisés pour lutter contre les insectes et autres parasites du sol, par exemple les coleopteres, comme DIABROTICA, les taupins et les vers blancs, les myriapodes comme les scutigérelles et les blaniules, et les diptères comme les cecydomies et les lepidopteres comme les noctuelles terricoles.

Ils sont utilisés à des doses comprises entre 10 g et 300 g de matière active à l'hectare.

Les produits de formule I peuvent également être utilisés pour lutter contre les insectes dans les locaux, pour lutter notamment contre les mouches, les moustiques et les blattes.

Les produits de formule I sont de plus photostables et sont peu toxiques pour les mammifères.

L'ensemble de ces propriétés fait des produits de formule des produits qui correspondent parfaitement aux exigences de l'industrie agrochimique moderne : ils permettent de protéger les récoltes tout en préservant l'environnement.

Les produits de formule I peuvent aussi être utilisés pour lutter contre les acariens et les nématodes parasites des végétaux.

Les composés de formule I peuvent encore être utilisés pour lutter contre les acariens parasites des animaux, pour lutter par exemple contre les tiques et notamment les tiques du genre de Boophilus, ceux du genre Hyalomnia, ceux du genre Amblyomnia et ceux du genre Rhipicephalus ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits de formule I définis ci-dessus et notamment les produits des exemples 1, 2, 3, 4, 5, 6, 10, 44 et 45.

L'invention a notamment pour objet les compositions insecticides renfermant comme principe actif au moins l'un des produits définis ci-dessus.

Ces compositions sont préparées selon les procédés usuels de l'industrie agrochimique ou de l'industrie vétérinaire ou de l'industrie des produits destinés à la nutrition animale.

Dans ces compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensioactif, non ionique, assurant, en outre, une dispersion uniforme des substances constitutives du mélange. Le véhicule utilisé peut être un liquide, tel que l'éau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

L'invention a également pour objet les compositions acaricides renfermant comme principe actif au moins un des produits de formule I définie ci-dessus.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % ou des liquides pour pulvérisation foliaire contenant de 1 à 500 g/l de principe actif. On peut également employer des poudres pour poudrages foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo /2,2-1/ 5-heptène-2,3-dicarboximide, ou le pipéronyl-bis-2-(2′-n-butoxy éthoxy) éthylacétal (ou tropital).

Les composés de formule I présentent une excellente tolérance générale, et l'invention a donc également pour objet les produits de formule I, pour lutter notamment contre les affections créées par les tiques et les gales chez l'homme et l'animal.

Les produits de l'invention sont notamment utilisés pour lutter contre les poux à titre préventif ou curatif et pour lutter contre la gale.

Les produits de l'invention peuvent être administrés par voie externe, par vaporisation, par shampooing, par bain ou badigeonnage.

Les produits de l'invention à usage vétérinaire peuvent être également administrés par badigeonnage de l'épine dorsale selon la méthode dite méthode "pour-on".

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale I, et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcools 3-phénoxy benzylique et d'alcools alpha-cyano 3-phénoxy benzyliques des acides chrysanthémiques, par les esters d'alcools 5- benzyl 3-furyl méthylique des acides 2,2-diméthyl 3-(2-oxo 3-tétrahydrothiophénylidène méthyl) cyclopropane-1-carboxyliques, par les esters d'alcool 3- phénoxy benzylique et d'alcools alpha-cyano 3-phénoxy benzyliques des acides 2,2-diméthyl 3-(2,2-dichlorovinyl) cyclopropane-1-carboxyliques, par les esters d'alcools alpha-cyano 3-phénoxy benzyliques d'acides 2,2-diméthyl 3- (2,2-dibromovinyl) cyclopropane-1-carboxyliques, par les esters d'alcools 3-phénoxy benzylique des acides 2-parachlorophényl 2-isopropyl acétiques, par les esters d'alléthrolones, d'alcool 3,4,5,6-tétrahydrophtalimidométhylique, d'alcool 5-benzyl 3-furyl méthylique, d'alcool 3-phénoxy benzylique et d'alcools alpha-cyano 3-phénoxy benzyliques des acides 2,2-diméthyl 3- (1,2,2,2-tétrahaloéthyl) cyclopropane--1-carboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

Les exemples suivants illustrent l'invention sans toutefois la limiter.

**Exemple 1 : [1R[1alpha,3alpha(E+Z)>] 3-(2-chloro 2-fluoro éthényl) 2,2-diméthyl cyclopropane carboxylate de 2,3,4,5,6-pentafluorophényl méthyle.**

On introduit à 0-5° C, une solution renfermant 2 g de dicyclohexylcarbo-diimide, 0,1 g de diméthylaminopyridine et 10 cm3 de chlorure de méthylène, dans une solution renfermant 1,75 g d'acide 1R-[1alpha,3alpha(E+Z)] 3-(2-chloro 2-fluoro éthényl 2,2-diméthyl cyclopropane carboxylique (préparé selon le procédé décrit à l'exemple 16 du brevet français 2396006), 2 g d'alcool pentafluorobenzylique et 80 $cm^3$ de chlorure de méthylène. On agite 15 minutes à 0° C puis 2 heures à 20° C. On filtre, concentre, reprend à l'éther isopropylique, glace, filtre et concentre. On chromatographie le produit obtenu sur silice en éluant par le mélange hexane éther isopropylique 95-5. On élimine le solvant et sèche. On obtient 2,85 g de produit recherché.
$alpha_D$ = 7°2 ± 1°5 (c=0,4 % $CHCl_3$).

**Exemples 2 à 6 :**

En opérant comme à l'exemple 1, à partir du même acide et des différents alcools concernés, on a préparé les produits suivants :

**Exemple 2 : [1R[1alpha,3alpha(E+Z)]] 3(2-chloro 2-fluoro éthényl) 2,2-diméthyl cyclopropane carboxylate de 2,3,5,6 tétrafluoro phényl méthyle.**

$alpha_D$ = +1°5 ± 2° (c=0,7 % $CHCl_3$).

**Exemple 3 : [1R[1alpha,3alpha(E+Z)]] 3(2-chloro 2-fluoro éthényl) 2,2-diméthyl cyclopropane carboxylate de 4-méthyl 2,3,5,6 tétrafluoro phénylméthyle.**

$alpha_D$ = +5°5 ± 2° (c=0,55 % $CHCl_3$).

**Exemple 4 : [1R[1alpha,3alpha(E+Z)]] 3(2-chloro 2-fluoro éthénylthyl cyclopropane carboxylate de 4-méthoxy 2,3,5,6tétrafluoro phénylméthyle.**

$alpha_D$ = +6° ± 2° (c=0,5 % $CHCl_3$).

**Exemple 5 : [1R[1alpha,3alpha(E+Z)]] 3(2-chloro 2-fluoro éthényl) 2,2-diméthyl cyclopropane carboxylate de 4-(2-propynyl) 2,3,5,6-tétrafluorophényl méthyle.**

$alpha_D$ = +11° ± 1° (c=0,5 % toluène).

**Exemple 6 : [1R[1alpha,3alpha(E+Z)]] 3(2-chloro 2-fluoro éthényl)2,2-diméthyl cyclopropane carboxylate de 4-(2-propényl) 2-,3,5,6-tétrafluorophényl méthyle.**

$alpha_D$ = +11° ± 1° (c=0,9 % toluène).

Les exemples 7 à 9 suivants ont été préparés à partir de l'acide (1R,cis) 2,2-diméthyl 3-(2-fluoro 2-bromoéthényl) cyclopropane carboxylique dont la préparation est donnée ci-après et des alcools correspondants :

**Exemple 7 : [1R(1alpha,3alpha(E+Z)] 2,2-diméthyl 3(2-fluoro-2-bromoéthényl) cyclopropane carboxylate de 2,3,4,5,6 pentafluorophényl méthyle.**

rf=0,17 $SiO_2$ flugène 113 pur.
$alpha_D$ = +11,5° ± 1,5° (c=0,7 % $CHCl_3$).

**Exemple 8 : [1R(1alpha,3alpha(E+Z)] 2,2-diméthyl 3(2-fluoro 2-bromoéthényl) cyclopropane carboxylate de 2,3,5,6 tétrafluorophényl méthyle.**

rf-0,15 $SiO_2$ flugène 113 pur.
$alpha_D$ = + 18° ± 1,5° (c=0,7 % $CHCl_3$).

**Exemple 9 : [1R(1alpha,3alpha(E+Z)] 2,2-diméthyl 3(2-fluoro 2-bromoéthényl) cyclopropane car-**

boxylate de 4-méthyl 2,3,5,6 tétrafluoro phénylméthyle.

rf=0,1 $SiO_2$ flugène 113 pur. $alpha_D$ = + 7,5° ± 1° (c=0,9 % $CHCl_3$).

**Exemples 10 à 33 :**

En opérant comme à l'exemple 1, à partir du même acide et des différents alcools appropriés, on a préparé les produits suivants :

**Exemple 10 : [1R [1alpha, 3-alpha (E + Z)]] 3-(2-chloro 2-fluoroéthényl) 2,2-diméthyl cyclopropane carboxylate de 4-éthyl 2,3,5,6-tétrafluorophénylméthyle.**

$[alpha]_D$ = +27,5° ± 2,5° (c = 0,3% $CHCl_3$)

**Exemple 11 : [1R [1alpha, 3-alpha (E + Z)]] 3-(2-chloro 2-fluoroéthényl) 2,2-diméthyl cyclopropane carboxylate de 4-triméthylsilyl 2,3,5,6-tétrafluorophénylméthyle.**

$[alpha]_D$ = +8° ± 2,5° (c = 0,2% toluène)

**Exemple 12 : [1R [1alpha, 3-alpha (E + Z)]] 3-(2-chloro 2-fluoroéthényl) 2,2-diméthyl cyclopropane carboxylate de 4- trifluorométhyl 2,3,5,6-tétrafluorophénylméthyle.**

$[alpha]_D$ = +9,5° ± 2° (c = 0,5% chloroforme)

**Exemple 13 : [1R [1alpha, 3-alpha (E + Z)]] 3-(2-chloro 2-fluoroéthényl) 2,2-diméthyl cyclopropane carboxylate de 4-difluorométhoxy 2,3,5,6-tétrafluorophénylméthyle.**

$[alpha]_D$ = +7° ± 2° (c = 0,5% toluène)

**Exemple 14 : [1R [1alpha, 3-alpha (E + Z)]] 3-(2-chloro 2-fluoroéthényl) 2,2-diméthyl cyclopropane carboxylate de 4-hydroxyméthylène 2,3,5,6-tétrafluorophénylméthyle.**

$[alpha]_D$ = +5,5° ± 1° (c = 1% $CHCl_3$)

**Exemple 15 : [1R [1alpha, 3-alpha (E + Z)]] 3-(2-chloro 2-fluoroéthényl) 2,2-diméthyl cyclopropane carboxylate de 4-(1-hydroxyéthyl) 2,3,5,6-tétrafluorophénylméthyle.**

$[alpha]_D$ = +4,5° ± 1° (c = 1% chloroforme)

**Exemple 16 : [1R [1alpha, 3-alpha (E + Z)]] 3-(2-chloro 2-fluoroéthényl) 2,2-diméthyl cyclopropane carboxylate de 4-acétoxyméthyl 2,3,5,6-tétrafluorophénylméthyle.**

$[alpha]_D$ = +3° ± 1,5° (c = 0,6% chloroforme)

**Exemple 17 : [1R [1alpha, 3-alpha (E + Z)]] 3-(2-chloro 2-fluoroéthényl) 2,2-diméthyl cyclopropane carboxylate de 4-tétrahydropyranyloxy 2,3,5,6-tétrafluorophénylméthyle.**

$[alpha]_D$ = +5,5° ± 2° (c = 0,5% $CHCl_3$) F = 66° C.

**Exemple 18 : [1R [1alpha, 3-alpha (E + Z)]] 3-(2-chloro 2-fluoroéthényl) 2,2-diméthyl cyclopropane carboxylate de 4-méthoxyméthyl 2,3,5,6-tétrafluorophénylméthyle.**

$[alpha]_D$ = +3° ± 1,5° (c = 0,7% $CHCl_3$)

**Exemple 19 : [1R [1alpha, 3-alpha (E + Z)]] 3-(2-chloro 2-fluoroéthényl) 2,2-diméthyl cyclopropane carboxylate de 4-(1-méthoxyéthyl) 2,3,5,6-tétrafluorophénylméthyle.**

[alpha]$_D$ = +10° ± 2° (c = 0,5% $CHCl_3$)

**Exemple 20 : [1R [1alpha, 3-alpha (E + Z)]] 3-(2-chloro 2-fluoroéthényl) 2,2-diméthyl cyclopropane carboxylate de 4-éthylèn 2,3,5,6-tétrafluorophénylméthyle.**

[alpha]$_D$ = +2,5° ± 1° (c = 0,8% $CHCl_3$)

**Exemple 21 : [1R [1alpha, 3-alpha (E + Z)]] 3-(2-chloro 2-fluoroéthényl) 2,2-diméthyl cyclopropane carboxylate de 4-butynyl 2,3,5,6-tétrafluorophénylméthyle.**

[alpha]$_D$ = +2° ± 0,5° (c = 1,3% $CHCl_3$) F = 86°C.

**Exemple 22 : [1R [1alpha, 3-alpha (E + Z)]] 3-(2-chloro 2-fluoroéthényl 2,2-diméthyl cyclopropane carboxylate de 4-azido 2,3,5,6-tétrafluorophénylméthyle.**

[alpha$_D$] = +19,5° ± 1,5° (c = 0,8% chloroforme)

**Exemple 23 : [1R [1alpha, 3-alpha (E + Z)]] 3-(2-chloro 2-fluoroéthényl 2,2-diméthyl cyclopropane carboxylate de 4-fluorophénylméhyle.**

[alpha]$_D$ = +10° ± 1° (c = 0,9% toluène)

**Exemple 24 : [1R [1alpha, 3-alpha (E + Z)]] 3-(2-chloro 2-fluoroéthényl 2,2-diméthyl cyclopropane carboxylate de 2,6-difluorophénylméhyle.**

[alpha]$_D$ = -9,5° ± 1,5° (c = 0,75% $CHCl_3$)

**Exemple 25 : [1R [1alpha, 3-alpha (E + Z)]] 3-(2-chloro 2-fluoroéthényl 2,2-diméthyl cyclopropane carboxylate de 2,5-difluorophénylméhyle.**

[alpha]$_D$ = +11,5° ± 2° (c = 0,45% toluène)

**Exemple 26 : [1R [1alpha, 3-alpha (E + Z)]] 3-(2-chloro 2-fluoroéthényl 2,2-diméthyl cyclopropane carboxylate de 2,3-difluorophénylméhyle.**

[alpha]$_D$ = +6,5° ± 1,5° (c = 0,8% $CHCl_3$)

**Exemple 27 : [1R [1alpha, 3-alpha (E + Z)]] 3-(2-chloro 2-fluoroéthényl 2,2-diméthyl cyclopropane carboxylate de 3,4-difluorophénylméhyle.**

[alpha]$_D$ = +16,5° ± 1° (c = 0,65% chloroforme)

**Exemple 28 : [1R [1alpha, 3-alpha (E + Z)]] 3-(2-chloro 2-fluoroéthényl 2,2-diméthyl cyclopropane carboxylate de 3,5-difluorophénylméhyle.**

[alpha]$_D$ = +21° ± 1,5° (c = 0,8% toluène)

**Exemple 29 : [1R [1alpha, 3-alpha (E + Z)]] 3-(2-chloro 2-fluoroéthényl 2,2-diméthyl cyclopropane carboxylate de 2,3,6-trifluorophénylméhyle.**

[alpha]$_D$ = +3° ± 2° (c = 0,5% toluène)

**Exemple 30 : [1R [1alpha, 3-alpha (E + Z)]] 3-(2-chloro 2-fluoroéthényl 2,2-diméthyl cyclopropane carboxylate de 2,4,5-trifluorophénylméhyle.**

[alpha]$_D$ = +2,5° ± 2° (c = 0,4% toluène)

**Exemple 31 : [1R [1alpha, 3-alpha (E + Z)]] 3-(2-chloro 2-fluoroéthényl 2,2-diméthyl cyclopropane**

carboxylate de 2,4,6- trifluorophénylméhyle.

$[alpha]_D$ = +2,5° (toluène) Rf = 0,46 cyclohexane-éther isopropylique 95-5)

**Exemple 32 : [1R [1alpha, 3-alpha (E + Z)]] 3-(2-chloro 2-fluoroéthényl 2,2-diméthyl cyclopropane carboxylate de 3,4,5-trifluorophénylméhyle.**

$[alpha]_D$ = +16,5° ± 2° (c = 0,5% chloroforme)

**Exemple 33 : [1R [1alpha, 3-alpha (E + Z)]] 3-(2-chloro 2-fluoroéthényl 2,2-diméthyl cyclopropane carboxylate de 2,3,4,5-tétrafluorophénylméhyle.**

$[alpha]_D$ = +24,5° ± 2° (c = 0,4% toluène)

**Exemples 34 à 41 :**

En opérant comme à l'exemple 1 à partir de l'acide 1R,trans (E + Z) 3-(2-chloro 2-fluoroéthényl) 2,2-diméthyl cyclopropane carboxylique (préparation 2) et des alcools appropriés, on a préparé les produits suivants :

**Exemple 34 : [1R,trans (E + Z)] 3-(2-chloro 2-fluoroéthényl 2,2-diméthyl cyclopropane carboxylate de 2,3,5,6-tétrafluorophénylméhyle.**

$[alpha]_D$ = +26° ± 1,5° (c = 0,65% chloroforme)

**Exemple 35 : [1R,trans (E + Z)] 3-(2-chloro 2-fluoroéthényl 2,2-diméthyl cyclopropane carboxylate de 2,3,4,5,6-pentafluorophénylméhyle.**

$[alpha]_D$ = -17° ± 2° (c = 0,45% chloroforme)

**Exemple 36 : [1R,trans (E + Z)] 3-(2-chloro 2-fluoroéthényl 2,2-diméthyl cyclopropane carboxylate de 4-méthyl 2,3,5,6-tétrafluorophénylméhyle.**

$[alpha]_D$ = -15° ± 2° (c = 0,4% chloroforme)

**Exemple 37 : [1R,trans (E + Z)] 3-(2-chloro 2-fluoroéthényl 2,2-diméthyl cyclopropane carboxylate de 4-méthoxy 2,3,5,6-tétrafluorophénylméhyle.**

$[alpha]_D$ = -28,5° ± 2,5° (c = 0,6% toluène)

**Exemple 38 : [1R,trans (E + Z)] 3-(2-chloro 2-fluoroéthényl 2,2-diméthyl cyclopropane carboxylate de 4-méthoxyméthyl 2,3,5,6-tétrafluorophénylméhyle.**

$[alpha]_D$ = -21° ± 2° (c = 0,5% chloroforme)

**Exemple 39 : [1R,trans (E + Z)] 3-(2-chloro 2-fluoroéthényl 2,2-diméthyl cyclopropane carboxylate de 4-éthynyl 2,3,5,6- tétrafluorophénylméhyle.**

$[alpha]_D$ = -40° ± 2,5° (c = 0,5% toluène)

**Exemple 40 : [1R,trans (E + Z)] 3-(2-chloro 2-fluoroéthényl 2,2-diméthyl cyclopropane carboxylate de 4-(2-propényl) 2,3,5,6-tétrafluorophénylméhyle.**

$[alpha]_D$ = -27° ± 1,5° (c = 1% toluène) F = 43,5° C.

**Exemple 41 : [1R,trans (E + Z)] 3-(2-chloro 2-fluoroéthényl 2,2-diméthyl cyclopropane carboxylate de 4-(2-propynyl) 2,3,5,6-tétrafluorophénylméhyle.**

[alpha]$_D$ = -24,5° ± 1° (c = 0,9% toluène)

Exemples 42 à 45 :

En opérant comme à l'exemple 1 à partir de l'acide utilisé à l'exemple 7 et des alcools appropriés, on a préparé les produits suivants :

**Exemple 42 : 1R,cis (E + Z) 3-(2-fluoro 2-bromoéthényl) 2,2-diméthyl cyclopropane carboxylate de 4-méthoxy 2,3,5,6-tétrafluorophénylméthyle.**

[alpha]$_D$ = +1,5° ± 1,5° (c = 0,75% $CHCl_3$)

**Exemple 43 : 1R,cis (E + Z) 3-(2-fluoro 2-bromoéthényl) 2,2-diméthyl cyclopropane carboxylate de 4-méthoxyméthyl 2,3,5,6-tétrafluorophénylméthyle.**

[alpha]$_D$ = +5° ± 1° (c = 1% chloroforme)

**Exemple 44 : 1R,cis (E + Z) 3-(2-fluoro 2-bromoéthényl) 2,2-diméthyl cyclopropane carboxylate de 4-(2-propényl) 2,3,5,6-tétrafluorophénylméthyle.**

CCM Rf = 0,25 (hexane-éther isopropylique 97-3).

**Exemple 45 : 1R,cis (E + Z) 3-(2-fluoro 2-bromoéthényl) 2,2-diméthyl cyclopropane carboxylate de 4-(2-propynyl) 2,3,5,6-tétrafluorophénylméthyle.**

CCM Rf = 0,22 (hexane-éther isopropylique 97-3).

Exemples 46 à 49 :

En opérant comme à l'exemple 1 à partir de l'acide 1R,trans (E + Z) 3-(2-fluoro 2-bromoéthényl) 2,2-diméthyl cyclopropane carboxylique (préparation 3) et avec les alcools appropriés, on a préparé les produits suivants :

**Exemple 46 : 1R,trans (E + Z) 3-(2-fluoro 2-bromoéthényl) 2,2-diméthyl cyclopropane carboxylate de 2,3,5,6-tétrafluorophénylméthyle.**

[alpha]$_D$ = -21° ± 2° (c = 0,5% chloroforme)

**Exemple 47 : 1R,trans (E + Z) 3-(2-fluoro 2-bromoéthényl) 2,2-diméthyl cyclopropane carboxylate de pentafluorophénylméthyle.**

[alpha]$_D$ = -21° ± 1,5° (c = 0,75% chloroforme)

**Exemple 48 : 1R,trans (E + Z) 3-(2-fluoro 2-bromoéthényl) 2,2-diméthyl cyclopropane carboxylate de 4-méthyl 2,3,5,6-tétrafluorophénylméthyle.**

[alpha]$_D$ = -21° ± 2° (c = 0,6% chloroforme)

**Example 49 : 1R,trans (E + Z) 3-(2-fluoro 2-bromoéthényl) 2,2-diméthyl cyclopropane carboxylate de 4-méthoxyméthyl 2,3,5,6-tétrafluorophénylméthyle.**

[alpha]$_D$ = -31° ± 2° (c = 0,6% chloroforme)

**Préparation 1: Acide (1Rcis) 2,2-diméthyl 3(2-fluoro 2-bromo éthényl) cyclopropane carboxylique.**

a) Condensation du dibromofluorométhane

On ajoute à -60°C en 20 minutes, une solution renfermant 10 g de terbutylate de potassium, 45 cm3 de terbutanol et 45 cm3 de tétrahydrofuranne, dans une solution renfermant 11,6 g de dibromofluorométhane, 100 cm3 de diméthyl formamide et 5,7 g de lactone de l'acide 1Rcis 2,2-diméthyl (dihydroxyméthyl) cyclopropane carboxylique. On verse sur une solution aqueuse d'acide chlorhydrique 2N et extrait au benzène. On lave à l'eau, sèche et amène à sec. On obtient 9,1 g de produit recherché.

b) Lactonisation

On ajoute au 9,1 g de produit préparé au stade (a), 100 cm3 de benzène anhydre, et 0,5 g d'acide paratoluène sulfonique. On porte la solution réactionnelle pendant 16 heures au reflux en éliminant l'eau formée. On laisse refroidir la solution obtenue à la température ambiante, la lave avec de la soude N puis à l'eau, la sèche et la concentre sous pression réduite. On obtient 6,3 g de produit recherché.

c) Elimination réductrice

On refroidit à +5°C une solution renfermant 6,3 g du produit préparé au stade précédent, 57 $cm^3$ d'acide acétique et 6,3 $cm^3$ d'eau. On ajoute à cette température 1,3 g de zinc en poudre fine. On agite le mélange réactionnel pendant 2 heures à 20°C, on filtre, rince à l'eau et au chlorure de méthylène. On décante, lave à l'eau et sèche le produit obtenu. On obtient 4,3 g d'une huile que l'on reprend à l'aide d'un mélange eau et glace et ajoute de la soude 2N jusqu'à pH 10. On lave au chlorure de méthylène et à l'essence G. On acidifie à pH3, extrait le précipité obtenu au chlorure de méthylène. On lave à l'eau, sèche et amène à sec. On obtient 3,1 g d'acide bromofluoré recherché.
RMN $CDCl_3$ ppm
H des $CH_3$ géminés 1,24 ppm
H acide 10,5 ppm
H en 1 et 3 du cyclopropane 1,7 à 2,1 ppm
Mélange E, Z 50/50.

**Préparation 2 : Acide 1R,trans (E + Z) 2,2-diméthyl 3-(2-fluoro 2-chloroéthényl) cyclopropane carboxylique.**

Stade A : (1R,trans) 3-diméthoxyméthyl 2,2-diméthyl cyclopropane carboxylate de méthyle.

On chauffe pendant 15 heures au reflux, 46,85 g de (1R,trans) 3-formyl 2,2-diméthyl cyclopropane carboxylate de méthyle, 47 $cm^3$ de méthanol, 0,47 g d'acide paratoluène sulfonique et 63,66 g d'orthoformiate de méthyle. On neutralise avec du bicarbonate de sodium et distille sous pression réduite. On obtient 56 g de produit recherché. Eb = 58°C sous 0,5 mm/Hg.

Spectre IR :

C=O : 1723 $cm^{-1}$
C-O-C : 1102 - 1052 $cm^{-1}$

Stade B : (1R,trans) 3-diméthoxyméthyl 2,2-diméthyl cyclopropane carboxylate de tert-butyle.

A un mélange de 51 g de terbutylate de potassium et 350 $cm^3$ de toluène, on ajoute 50 g du produit obtenu au stade A ci-dessus. On agite pendant 7 heures à 20/25°C. On verse dans 500 $cm^3$ d'eau et glace. On décante, lave à l'eau, sèche, filtre et évapore les solvants sous pression réduite. On distille le résidu sous pression réduite (Eb = 75°C sous 0,05 mm/Hg) et on obtient 54,93 g de produit recherché.

Spectre IR :

-C=O ester : 1711 $cm^{-1}$
géminés de $CH_3$ : 1395 = 1381 $cm^{-1}$
t-bu : 1370 $cm^{-1}$

Stade C : (1R,trans) 3-formyl 2,2-diméthyl cyclopropane carboxylate de terbutyle.

On agite pendant 15 heures à 20/25° C 54,93 g du produit obtenu au stade B, 500 $cm_3$ de diméthylformamide et 275 $cm_3$ d'acide chlorhyrique N. On verse le mélange réactionnel dans 50 $cm_3$ d'eau. On décante et extrait avec du benzène, sèche, filtre et distille sous pression réduite. On obtient 27,97 g de produit attendu. Eb = 81° C sous 1,5 mm/Hg.

Spectre IR :

max ester : 1705 $cm^{-1}$
épaulement : 1720 $cm^{-1}$
CH de l'aldéhyde : 2740 $cm^{-1}$

Stade D : (1R,trans) (E + Z) 2,2-diméthyl 3-(2-fluoro 2-chloroéthényl) cyclopropane carboxylate de tert-butyle.

A un mélange agité de 39,3 g de triphénylphosphine, 250 $cm_3$ d'heptane et 11,25 g de tert-butanol, on ajoute 17 g de tert-butylate de potassium. On agite 15 minutes, refroidit à 0° C et ajoute en 30 minutes une solution de 18 g de dichlorofluorométhane dans 200 $cm_3$ d'heptane. On laisse la température remonter à 15° C puis ajoute en 20 minutes une solution de 23,7 g du produit obtenu au stade C dans 25 $cm_3$ d'heptane. On chauffe 1 heure à 50/55° C sous agitation et laisse 16 heures à température ambiante. On filtre l'insoluble et évapore le solvant sous pression réduite. Le résidu (38 g) est chromatographié sur silice (éluant : benzène pur). On obtient 15,4 g de produit recherché. Rf = 0,5 éluant : benzène pur. Ce produit est utilisé tel quel pour le stade suivant.

Stade E : Acide 1R,trans (E + Z) 2,2-diméthyl 3-(2-fluoro-2-chloroéthényl) cyclopropane carboxylique.

On agite pendant 1 heure 30 minutes au reflux le mélange de 15,4 g du produit obtenu au stade C, 150 $cm_3$ de toluène et 800 mg d'acide paratoluènesulfonique. On amène ensuite à sec sous pression réduite. Le résidu (14 g) est chromatographié sur silice (éluant : benzène-acétate d'éthyle 1-1). On obtient 11,5 g du produit recherché.
Spectre RMN ($CDCl_3$) 60 MHz :
géminés diméthyl : 71-79 Hz
H de l'acide : 680 Hz

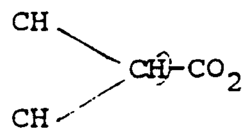 : 88-93,5 Hz

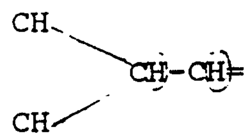 : 117 à 135 Hz

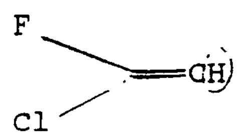

262-270 
289-297 } JH8 trans 27

et

298-306 
306-316 } JH8 cis ~ 8 à 9

(E + Z ~ 1/2 - 1/2).

**Préparation 3 : Acide (1R,trans) (E + Z) 2,2-diméthyl 3-(2-fluoro 2-bromoéthényl) cyclopropane carboxylique.**

Stade A: (1R,trans) (E + Z) 2,2-diméthyl 3-(2-fluoro 2-bromoéthényl) cyclopropane carboxylate de tert-butyle.

A une solution de 3,2 g de triphénylphosphine, 2,4 g de dibromofluorométhane, 100 mg de chlorure de benzyltriéthyl ammonium et 10 $cm_3$ de chlorure de méthylène, on ajoute sans dépasser 30° C, 10 $cm_3$ de soude 50%, on gite 30 minutes et ajoute 2g du produit obtenu au stade C de la préparation . On agite pendant 4 heures à température ambiante, décante la phase aqueuse et lave la phase organique, sèche, filtre et contretre à sec sous pression réduite. On empâte le résidu dans 2 volumes d'éther de pétrole (Eb : 60-80° C), filtre l'insoluble et concentre le filtrat à sec sous pression réduite et obtient 4 g d'huile que l'on chromatographie sur silice (éluant : cyclohexane-toluène 5-5). On obtient 2,06 g de produit recherché.
Spectre RMN ($CDCl_3$) 60 MHz :
géminés méthyl : 68-74-76 Hz
t-bu : 86,5 Hz

H de 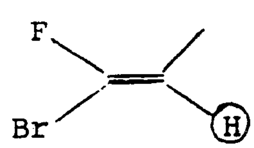 : 268 à 330 Hz

Delta E et delta Z.

Stade B : Acide (1R,trans) (E + Z) 2,2-diméthyl 3-(2-fluoro 2-bromoéthényl) cyclopropane carboxylique.

On opère comme au stade E de la préparation 2 à partir de 1,5 g du produit obtenu au stade A ci-dessus. On obtient 1,03 g de produit attendu.
Spectre RMN ($CDCl_3$) 60 MHz :
méthyl géminés : 70-77-79 Hz

H de COOH : 538 Hz

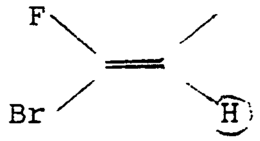 : 270 à 332 Hz

Delta E + delta Z = 1/2 + 1/2.

**Exemple 50 : Préparation d'un concentré soluble**

On effectue un mélange homogène de :
Produit de l'exemple 1 ou 45 : 0,25 g
Butoxyde pipéronyle : 1,00 g
Tween 80 : 0,25 g
Topanol A : 0,1 g
Eau : 98,4 g

**Exemple 51 : Préparation d'un concentré émulsifiable**

On mélange intimement :
Produit de l'exemple 2 ou 10 : 0,015 g
Butoxyde de pipéronyle : 0,5 g
Topanol A : 0,1 g
Tween 80 : 3,5 g
Xylène : 95,885 g

**Exemple 52 : Préparation d'un concentré émulsifiable**

On effectue un mélange homogène de :
Produit de l'exemple 1 ou 45 : 1,5 g
Tween 80 : 20,00 g
Topanol A : 0,1 g
Xylène : 78,4 g

**Exemple 53 : Préparation d'une composition fumigène**

On mélange d'une façon homogène :
Produit de l'exemple 5 : 0,25 g
Poudre de tabu : 25,00 g
Poudre de feuille de cèdre : 40,00 g
Poudre de bois de pin : 33,75 g
Vert brillant : 0,5 g
p-Nitrophénol : 0,5 g

## ETUDE BIOLOGIQUE

A. Etude de l'effet d'abattage sur mouche domestique

Les insectes tests sont des mouches domestiques femelles âgées de 4 jours. On opère par pulvérisation directe à la concentration de 0,25 g/l en chambre de Kearns et March en utilisant comme solvant un mélange d'acétone (5 %) et d'Isopar L (solvant pétrolier) (quantité de solvant utilisée 2 ml en une seconde). On utilise 50 insectes par traitement. On effectue les contrôles toutes les minutes jusqu'à 10 minutes, puis à 15 minutes et l'on détermine le KT 50 par les méthodes habituelles.

Les résultats expérimentaux obtenus sont résumés dans le tableau suivant :

| Composés | KT50 en mn | Concentration en g/l |
|---|---|---|
| Exemple 1 | 2,3 | 1 |
| Exemple 2 | 2,4 | 1 |
| Exemple 5 | 3,2 | 0,1 |
| Exemple 13 | 7,1 | 0,1 |
| Exemple 18 | 2,9 | 0,1 |
| Exemple 20 | 9,4 | 0,1 |
| Exemple 29 | 1,3 | 1 |
| Exemple 34 | 5,7 | 0,1 |
| Exemple 36 | 1,8 | 1 |
| Exemple 38 | 2,2 | 0,1 |
| Exemple 42 | 6,2 | 0,1 |
| Exemple 43 | 3,5 | 0,1 |
| Exemple 45 | 2,2 | 0,1 |
| Exemple 47 | 9,4 | 0,1 |
| Exemple 49 | 3,6 | 0,1 |

B. Etude de l'effet létal des composés de l'invention sur divers insectes

a) Etude de l'effet létal sur mouche domestique

Les insectes tests sont des mouches domestiques femelles âgées de 4 à 5 jours. On opère par application topique de 1 microlitre de solution acétonique sur le thorax dorsal des insectes à l'aide du micro manipulateur d'Arnold. On utilise 50 individus par traitement. On effectue le contrôle de mortalité vingt-quatre heures après traitement.

Les résultats obtenus exprimés en $DL_{50}$ ou dose (en nanogrammes) par individu nécessaire pour tuer 50 % des insectes, sont les suivants :

| Composés de l'exemple | DL 50 en ng/insecte |
|---|---|
| 5 | 3,8 |
| 6 | 2,3 |
| 38 | 4,9 |
| 44 | 4,7 |
| 45 | 2,1 |

b) Etude de l'effet létal sur larves de Spodoptera Littoralis

Les essais sont effectués par application topique d'une solution acétonique à l'aide du micro manipulateur d'Arnold sur le thorax dorsal des larves. On utilise 15 larves par dose de produit à tester. Les larves utilisées sont des larves du quatrième stade larvaire, c'est-à-dire âgées d'environ 10 jours lorsqu'elles sont

élevées à 24 °C et 65 % d'humidité relative. Après traitement les individus sont placés sur milieu nutritif artificiel (milieu de Poitout).

On effectue le contrôle des mortalités 48 heures après traitement.

Les résultats expérimentaux obtenus sont résumés dans le tableau suivant :

| Composés de l'exemple | DL 50 en ng par insecte |
|---|---|
| 5 | 1,1 |
| 6 | 2,8 |
| 18 | 2,3 |
| 38 | 4,6 |
| 44 | 4,6 |
| 45 | 2,1 |

c) Etude de l'activité sur Diabrotica

Les insectes tests sont des larves de dernier stade de Diabrotica.

On traite une rondelle de papier filtre de 9 cm de diamètre, déposée au fond d'une boite de Petri, à l'aide de 2 $cm^3$ de solution acétonique. Après séchage on dépose 10 larves par dose et on effectue le contrôle de mortalité 24 h après le traitement.

On détermine la dose létale 100 (DL 100) exprimée en mg/litre.

Les résultats obtenus sont les suivants :

Produit de l'exemple 1 :
Produit de l'exemple 2 :
Produit de l'exemple 3 : 0,6
Produit de l'exemple 4 : 0,6
Produit de l'exemple 5 :
Produit de l'exemple 6 : 0,3
Produit de l'exemple 10 : 0,07
Produit de l'exemple 18 : 0,3
Produit de l'exemple 29 : 1,2
Produit de l'exemple 44 : 0,15
Produit de l'exemple 45 : 0,04

C) ETUDE ACARICIDE DES COMPOSES DE L'INVENTION

On utilise des plants de haricot comportant 2 feuilles, infestées de 25 femelles de Tetranychus Urticae par feuille et mis sous bonette aérée sous plafond lumineux en lumière constante. Les plants sont traités au pistolet Fischer : 4 ml de solution toxique par plant d'un mélange à volume égal d'eau et d'acétone. On laisse sécher pendant 12 heures puis on procède à l'infestation. Les contrôles de mortalité sont effectués 80 heures après. La dose utilisée est de 5 g de produit par hl. On détermine la concentration létale 50 (CL 50).

Les résultats expérimentaux sont les suivants :

| Composés de l'exemple | CL 50 en mg/l |
|---|---|
| 5 | 682 |
| 6 | 1357 |
| 44 | 843 |
| 45 | 429 |

D) Etude de l'effet létal sur Aphis cracivora.

On utilise des adultes après 7 jouts et l'on emploie 10 Aphis par concentration utilisée. On utilise une méthode de contact-injection. On effectue le traitement au pistolet de Fisher d'une feuille de fève que l'on dépose dans une boîte de Pétri en matière plastique sur une rondelle de papier humidifiée. Le traitement est effectué à l'aide de 2 ml de solution acétonique de produit à tester (1ml par face de feuille). L'infestation par insecte est effectuée après séchage de la feuille. On maintient les insectes en contact avec la feuille pendant une heure. On place les insectes sur des feuilles non traitées et contrôle la mortalité au bout de 24 heures.

Les résultats expérimentaux obtenus sont résumés dans le tableau suivant :

| Composés de l'exemple | CL 50 en mg/l |
|---|---|
| 5 | 0,01 |
| 6 | 0,10 |
| 10 | 0,7 |
| 18 | 0,2 |
| 38 | 2,1 |
| 43 | 0,4 |
| 44 | 0,04 |
| 45 | 0,004 |
| 49 | 0,7 |

**Revendications**

1) Sous toutes les formes stéréoisomères possibles ainsi que les mélanges de ces stéréoisomères les composés de formule (I) :

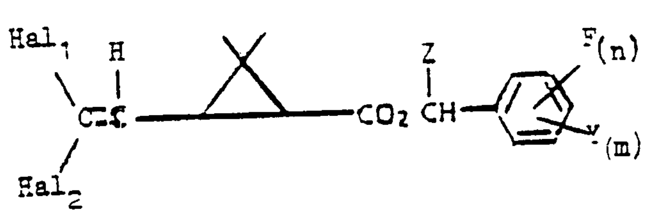

(I)

dans laquelle :

- $Hal_1$ et $Hal_2$ différents l'un de l'autre représentent un atome de fluor, de chlore, de brome ou l'iode,
- Z représente un atome d'hydrogène, un radical $CH_3$, C≡N ou C≡CH,
- n représente un nombre entier pouvant varier de 1 à 5,

- m représente le nombre 5-n et,
- Y représente un atome d'hydrogène, un atome d'halogène, un radical $CH_2$-C≡N, un radical hydroxy, un radical alkyle linéaire, ramifié, saturé ou insaturé éventuellement substitué renfermant jusqu'à 8 atomes de carbone un radical nitrile, un radical

$\overset{O}{\overset{\|}{C}}$ -O alkyle,

$\overset{O}{\overset{\|}{C}}$ -alkyle, $(CH_2)m'$ O-alkyle, $(CH_2)m'$ S-alkyle, $(CH_2)m'$ N-$(alkyle)_2$, dans lequel m' représente le nombre 0, 1, 2, 3 ou 4 et renfermant jusqu'à 12 atomes de carbone, un radical Si $(alkyle)_3$, alkyle représente un radical alkyle linéaire ou ramifié, saturé ou insaturé, éventuellement substitué renfermant jusqu'à 8 atomes de carbone, un radical O-aryle ou -$(CH_2)m'$-aryle, aryle représentant un radical aryle renfermant jusqu'à 14 atomes de carbone, le ou les substituants Y identiques ou différents, pouvant être en position quelconque sur le noyau phényle.

2) Les composés de formule (I) tels que définis à la revendication 1 répondant à la formule $(I_A)$ :

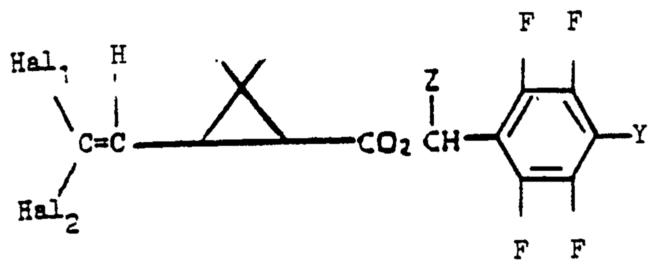

$(I_A)$

dans lesquels $Hal_1$, $Hal_2$, Z et Y conservent la même signification que dans la revendication 1.

3) Les composés de formule $(I_A)$ tels que définis à la revendication 2 dans lesquels :
- Y représente un atome d'hydrogène, un atome d'halogène, un radical hydroxy, un radical alkyle linéaire, ramifié, saturé ou insaturé éventuellement substitué renfermant jusqu'à 8 atomes de carbone, un radical nitrile, un radical $(CH_2)m'$ O-alkyle, $(CH_2)m'$ S alkyle, $(CH_2)m'$ N-$(alkyle)_2$, dans lequel m représente le nombre 0,1,2,3 ou 4 et renfermant jusqu'à 12 atomes de carbone un radical Si $(alkyle)_3$, alkyle représente un radical alkyle linéaire ou ramifié, saturé ou insaturé, éventuellement substitué renfermant jusqu'à 8 atomes de carbone, un radical O-aryle ou -$(CH_2)m'$-aryle, aryle représentant un radical aryle renfermant jusqu'à 14 atomes de carbone.

4) Les composés de formule (I) ou $(I_A)$ tels que définis à l'une quelconque des revendications 1 à 3 dans lesquels $Hal_1$ et $Hal_2$ représentent l'un un atome de fluor, et l'autre un atome de chlore.

5) Les composés de formule (I) ou $(I_A)$ tels que définis à l'une quelconque des revendications 1 à 3 dans lesquels $Hal_1$ et $Hal_2$ représentent l'un un atome de fluor, et l'autre un atome de brome.

6) Les composés de formule (I) ou $(I_A)$ tels que définis à l'une quelconque des revendications 1 à 5 dans lesquels Z représente un atome d'hydrogène.

7) Les composés de formule (I) ou $(I_A)$ tels que définis à l'une quelconque des revendications 1 à 6 dans lesquels Y représente un atome de fluor.

8) Les composés de formule (I) ou $(I_A)$ tels que définis à l'une quelconque des revendications 1 à 6 dans lesquels Y représente un atome d'hydrogène.

9) Les composés de formule (I) ou $(I_A)$ tels que définis à l'une quelconque des revendications 1 à 6 dans lesquels Y représente un radical 2-propynyle.

10) Les composés de formule (I) ou $(I_A)$ tels que définis à l'une quelconque des revendications 1 à 6 dans lesquels Y représente un radical 2-propényle.

11) Les composés de formule (I) ou $(I_A)$ tels que définis à l'une quelconque des revendications 1 à 6 dans lesquels Y représente un radical éthyle.

12) Les composés de formule générale (I) ou $(I_A)$ tels que définis à l'une quelconque des revendications 1 à 11 dans lesquels la copule cyclopropanique est de structure 1R,cis.

13) Les composés de formule (I) ou $(I_A)$ tels que définis à la revendication 12 dont les noms suivent :
- [1R[1alpha,3alpha(E+Z)]] 3(2-chloro 2-fluoro éthényl) 2,2-diméthyl cyclopropane carboxylate de 2,3,4,5,6 pentafluoro phényl méthyle.
- [1R[1alpha,3alpha(E+Z)] 3(2-chloro 2-fluoro éthényl) 2,2-diméthyl cyclopropane carboxylate de 2,3,5,6 tétrafluoro phényl méthyle.
- [1R[1alpha,3alpha(E+Z)] 3(2-chloro 2-fluoro éthényl) 2,2-diméthyl cyclopropane carboxylate de 2,3,5,6

tétrafluoro 4-méthyl phényl méthyle.

- [1R(1alpha,3alpha(E+Z)]] 3(2-chloro 2-fluoro éthényl) 2,2--diméthyl cyclopropane carboxylate de 2,3,5,6 tétrafluoro 4-méthoxy phényl méthyle.
- [1R[1alpha,3alpha(E+Z)]] 3(2-chloro 2-fluoro éthényl) 2,2--diméthyl cyclopropane carboxylate de 2,3,5,6 tétrafluoro 4-(2-propynl) phénylméthyle.
- [1R[1alpha,3alpha(E+Z)]] 3(2-chloro 2-fluoro éthényl) 2,2-diméthylcyclopropane carboxylate de 2,3,5,6 tétrafluoro 4-(2-propényl) phényl méthyle.
- [1R[1alpha,3alpha(E+Z)]] 3(2-chloro 2-fluoro éthényl) 2,2-diméthylcyclopropane carboxylate de 4-éthyl de 2,3,5,6-tétrafluoro 4-(2-propényl) phényl méthyle.
- 1R,cis (E+Z) 3-(2-fluoro 2-bromoéthényl) 2,2-diméthyl cyclo propane carboxylate 4-(2-propényl 2,3,5,6-tétrafluorophényl méthyle.
- 1R,cis (E+Z) 3-(2-fluoro 2-bromoéthényl) 2,2-diméthyl cyclopropane carboxylate 4-(2-propynyl) 2,3,5,6-tétrafluorophényl méthyle.

14) Procédé de préparation des composés de formule (I) tels que définis à l'une quelconque des revendications 1 à 13 caractérisé en ce que l'on soumet un acide de formule (II) :

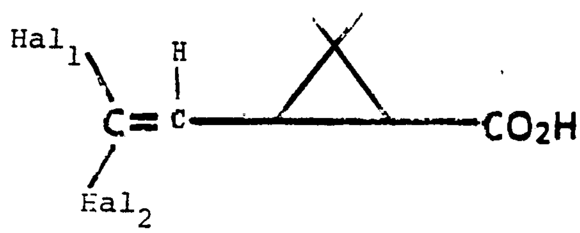

(II)

dans laquelle $Hal_1$ et $Hal_2$ conservent leur signification précédente à l'action d'un alcool (III) :

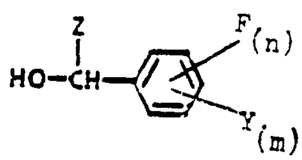

(III)

dans laquelle Y, Z, m et n conservent leur signification précédente pour obtenir le composé de formule (I) correspondant.

15) Application des composés de formule (I) ou ($I_A$) tels que définis à l'une quelconque des revendications 1 à 13, à la lutte contre les parasites des végétaux et les parasites des locaux.

16) Les compositions destinées à la lutte contre les parasites des végétaux, les parasites des locaux et les parasites des animaux à sang chaud, caractérisées en ce qu'elles renferment comme principe actif au moins un composé défini à l'une quelconque des revendications 1 à 13.

17) Les compositions insecticides, caractérisées en ce qu'elles renferment comme principe actif au moins un composé défini à l'une quelconque des revendications 1 à 13.

18) Les compositions insecticides définies à la revendication 17 caractérisées en ce qu'elles sont destinées à la lutte contre DIABROTICA et les autres parasites du sol.

19) Les compositions acaricides, caractérisées en ce qu'elles renferment comme principe actif au moins un composé défini à l'une quelconque des revendications 1 à 13.

20) Associations douées d'activité insecticide, acaricide ou nématicide, caractérisées en ce qu'elles contiennent comme matière active, d'une part, un au moins des composés de formule générale (I) et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3- tétrahydrothiophénylidène méthyl) cyclopropane-1-carboxyliques, par les esters d'alcool 3-phénoxy benzylique et d'alcools alpha-cyano 3-phénoxy benzyliques des acides 2,2-diméthyl 3-(2,2-dichlorovinyl) cyclopropane-1- carboxyliques, par les esters d'alcools alpha-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6tétrahydrophtalimidométhylique,

d'alcool 5benzyl 3furyl méthylique, d'alcool 3phénoxy benzylique et d'alcool alphacyano 3phénoxy benzyliques des acides 2,2diméthyl 3(-1,2,2,2-tétrahaloéthyl) cyclopropane-1-carboxyliques, dans lesquels "halo" représente un atome de fluor, de chlore ou de brome, étant entendu que les composés (I) peuvent exister sous toutes leurs formes stéréoisomères possibles de même que les copules acides et alcoools des esters pyréthrinoïdes ci-dessus.

21) A titre de produit chimique nouveau :
- l'acide (1Rcis) 2,2-diméthyl 3(2- fluoro 2-bromo éthényl) cyclopropane carboxylique,
- l'acide (1R,trans) 2,2-diméthyl 3-(2-fluoro 2-chloro éthényl) cyclopropane carboxylique,
- l'acide (1Rtrans) 2,2-diméthyl 3-(2-fluoro 2-bromoéthényl) cyclopropane carboxylique,

Revendications pour l'Etat contractant suivant : ES

1) Procédé de préparation des composés de formule (I) :

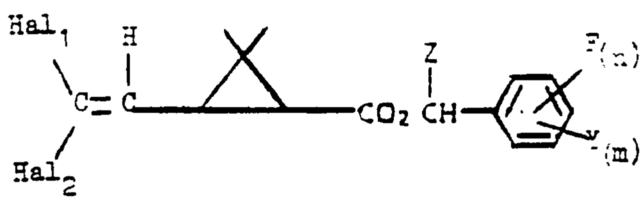

(I)

sous toutes les formes stéréoisomères possibles ainsi que les mélanges de ces stéréoisomères dans laquelle :
- $Hal_1$ et $Hal_2$ différents l'un de l'autre représentent un atome de fluor, de chlore, de brome ou d'iode,
- Z représente un atome d'hydrogène, un radical $CH_3$, C≡N ou C≡CH,
- n représente un nombre entier pouvant varier de 1 à 5,
- m représente le nombre 5-n et,
- Y représente un atome d'hydrogène, un atome d'halogène, un radical $CH_2$-C≡N, un radical hydroxy, un radical alkyle linéaire, ramifié, saturé ou insaturé éventuellement substitué renfermant jusqu'à 8 atomes de carbone un radical nitrile, un radical

$\overset{O}{\overset{\|}{C}}$ -O alkyle,

$\overset{O}{\overset{\|}{C}}$ -alkyle, $(CH_2)m'$ O-alkyle, $(CH_2)m'$ S-alkyle, $(CH_2)m'$ N-$(alkyle)_2$, dans lequel m' représente le nombre 0, 1, 2, 3 ou 4 et renfermant jusqu'à 12 atomes de carbone, un radical Si $(alkyle)_3$, alkyle représente un radical alkyle linéaire ou ramifié, saturé ou insaturé, éventuellement substitué renfermant jusqu'à 8 atomes de carbone, un radical O-aryle ou -$(CH_2)m'$-aryle, aryle représentant un radical aryle renfermant jusqu'à 14 atomes de carbone, le ou les substituants Y identiques ou différents, pouvant être en position quelconque sur le noyau phényle, caractérisé en ce que l'on soumet un acide de formule (II) :

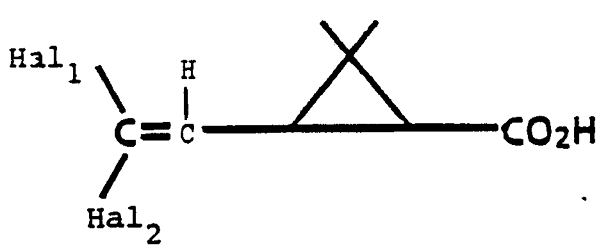

(II)

dans laquelle $Hal_1$ et $Hal_2$ conservent leur signification précédente à l'action d'un alcool (III) :

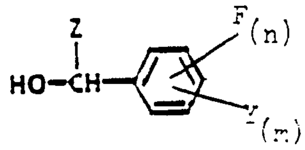

(III)

dans laquelle Y, Z, m et n conservent leur signification précédente pour obtenir le composé de formule (I) correspondant.

2) Procédé selon la revendication 1, caractérisé en ce que l'on utilise au départ le produit de formule (III) répondant à la formule :

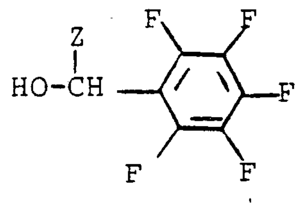

dans lesquels Z et Y conservent la même signification que dans la revendication 1.

3) Procédé selon la revendication 2, caractérisé en ce que dans le produit de formule (III) :

- Y représente un atome d'hydrogène, un atome d'halogène, un radical hydroxy, un radical alkyle linéaire, ramifié, saturé ou insaturé éventuellement substitué renfermant jusqu'à 8 atomes de carbone, un radical nitrile, un radical $(CH_2)m'$ O-alkyle, $(CH_2)m'$ S alkyle, $(CH_2)m'$ N-$(alkyle)_2$, dans lequel m représente le nombre 0,1,2,3, ou 4 et renfermant jusqu'à 12 atomes de carbone un radical Si $(alkyle)_3$, alkyle représente un radical alkyle linéaire ou ramifié, saturé ou insaturé, éventuellement substitué renfermant jusqu'à 8 atomes de carbone, un radical O-aryle ou -$(CH_2)m'$-aryle, aryle représentant un radical aryle renfermant jusqu'à 14 atomes de carbone.

4) Procédé selon l'une quelconque des revendications 1 à 3, caractérisé en que l'on utilise au départ un produit de formule (II) dans laquelle $Hal_1$ et $Hal_2$ représentent l'un un atome de fluor, et l'autre un atome de chlore.

5) Procédé selon l'une quelconque des revendications 1 à 3, caractérisé en que l'on utilise au départ un produit de formule (II) dans laquelle $Hal_1$ et $Hal_2$ représentent l'un un atome de fluor, et l'autre un atome de brome.

6) Procédé selon l'une quelconque des revendications 1 à 5, caractérisé en ce que l'on utilise au départ un produit de formule (III) dans laquelle Z représente un atome d'hydrogène.

7) Procédé selon l'une quelconque des revendications 1 à 6, caractérisé en ce que l'on utilise au départ un produit de formule (III) dans laquelle Y représente un atome de fluor.

8) Procédé selon l'une quelconque des revendications 1 à 6, caractérisé en ce que l'on utilise au départ un produit de formule (III) dans laquelle Y représente un atome d'hydrogène.

9) Procédé selon l'une quelconque des revendications 1 à 6, caractérisé en ce que l'on utilise au départ un produit de formule (III) dans laquelle Y représente un radical 2-propynyle.

10) Procédé selon l'une quelconque des revendications 1 à 6, caractérisé en ce que l'on utilise au départ un produit de formule (III) dans laquelle Y représente un radical 2-propényle.

11) Procédé selon l'une quelconque des revendications 1 à 6, caractérisé en ce que l'on utilise au départ un produit de formule (III) dans laquelle Y représente un radical éthyle.

12) Procédé selon l'une quelconque des revendications 1 à 11, caractérisé en ce que l'on utilise au départ un produit de formule (II) dans laquelle la copule cyclopropanique est de structure 1R,cis.

13) Procédé selon la revendication 2, caractérisé en ce que l'on utilise au départ l'acide [1R-[1alpha,3alpha(E+Z)]] 3-(2-chloro 2-fluoro éthényl) 2,2-diméthyl cyclopropane carboxylique ou l'acide 1R,cis (E+Z) 2,2-diméthyl 3-(2-fluoro 2-bromoéthényl) cyclopropane carboxylique et l'alcool pentafluorobenzylique, l'alcool 2,3,5,6-tétrafluorobenzylique, l'alcool 4-méthyl 2,3,5,6-tétrafluorobenzylique, l'alcool 4-méthoxy 2,3,5,6-tétrafluorobenzylique, l'alcool 4-(2-propényl) 2,3,5,6-tétrafluorobenzylique ou l'alcool 4-(2-propynyl) 2,3,5,6-tétrafluoronbenzylique.

Office européen des brevets

# RAPPORT DE RECHERCHE EUROPEENNE

Numero de la demande

EP 89 40 3669

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | GB-A-2 034 703 (SHELL INTERNATIONALE RESEARCH)<br>* Revendications; exemple 8; tableau I * | 1,4-7, 14-20 | C 07 C 69/743<br>A 01 N 53/00<br>C 07 C 61/40<br>C 07 F 7/08 |
| X | DE-A-2 941 332 (SHELL INTERNATIONALE RESEARCH)<br>* Revendications; exemple 8; tableau I * | 1,4-7, 14-20 | |
| X | CHEMICAL ABSTRACTS, TENTH COLLECTIVE INDEX, vols. 86-95, 1977-1981, Chemical Substances Copper(1+)-L-Cysteinamide, Seite 17558CS, Chemical Abstracts Service, Columbus, Ohio, US; "Cyclopropanecarboxylic acid, 3-(2-chloro-2-fluorethenyl)-2,2-dimethyl -(1R-trans)-[77789-64-1], prepn. and esterification of, by cyclopentenol deriv., 95:P 6614m"<br>* Page 17558CS, colonne 1, lignes 6,7,10,11 * | 21 | **DOMAINES TECHNIQUES RECHERCHES (Int. Cl.5)** |
| D,A | EP-A-0 031 199 (ICI)<br>* Revendications; pages 2-7 * | 1-20 | C 07 C 61/00<br>C 07 C 69/00 |
| A | EP-A-0 054 360 (ICI)<br>* Revendications; pages 2-5 * | 1-20 | |
| A | EP-A-0 000 229 (SHELL INTERNATIONALE RESEARCH)<br>* Revendications; pages 6-8 * | 1-21 | |

Le présent rapport a été établi pour toutes les revendications

| Lieu de la recherche | Date d'achèvement de la recherche | Examinateur |
|---|---|---|
| LA HAYE | 27-03-1990 | WRIGHT M.W. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)